# EUROPEAN PATENT SPECIFICATION

(11) **EP 3 995 826 B1**
(45) Date of publication and mention of the grant of the patent: **04.09.2024**
(21) Application number: 20834987.8
(22) Date of filing: 29.06.2020
(51) Int. Cl.: G01N 33/49, G01N 33/52, G01N 21/78, G01N 21/77, B01L 3/00, C12Q 1/54

(54) **BIOMATERIAL ASSAY STRIP**
BIOMATERIALTESTSTREIFEN
BANDE DE DOSAGE DE BIOMATÉRIAU

(30) Priority: 03.07.2019 KR 20190080231
(43) Date of publication of application: 11.05.2022
(73) Proprietor: 1Drop Inc., Gyeonggi-do 13217 (KR)
(72) Inventor: RHEE, Joo Won, Seongnam-si Gyeonggi-do 13562 (KR)
(74) Representative: advotec.
(86) International application number: PCT/KR2020/008433
(87) International publication number: WO 2021/002643

(56) References cited:
- EP-A2- 0 799 896
- EP-A2- 1 118 377
- WO-A1-2004/083852
- WO-A1-2019/135526
- JP-A- 2005 274 497
- KR-A- 20000 022 630
- KR-B1- 100 490 185
- KR-B1- 102 079 783

## Description

### [Technical Field]

The present invention relates to biomaterial measurement, and more specifically, to an assay strip for measuring a substance produced in the body using a body fluid.

### [Background Art]

In the current medical diagnosis field, various analysis techniques such as an immunoassay, a chemical colorimetric assay, and an electrochemical assay are used to detect or quantify specific substances contained in biological samples such as blood, serum, urine, cell fluid, and the like. These analysis techniques are being applied to large-scale equipment such as automatic analysis devices used in clinical centers of large hospitals or to test strips and cartridge devices used for point-of-care test (POCT) platforms.

When used in large-scale equipment, the analysis techniques have an advantage of being able to process a large number of samples quickly and obtaining measurement values with high reliability by converting the color value of a specific substance, which reacts with a liquid reagent, changed through an immune or enzymatic reaction to a concentration. However, due to the features of the equipment, the complicated structure and large size of a mechanical device allows it to be used only in a specific space, and thus there is a limitation in location. Also, it is inconvenient in terms of maintenance due to the pretreatment of blood such as centrifugation and periodic replacement of various types of reagents and sensors.

Meanwhile, POCT devices measure a specific substance through the same reaction principle as the large-scale equipment, but they use a strip or a cartridge in measurement, and thus measurement and management are easy. Also, rapid measurement is possible without restrictions on the measurement location, and thus the POCT devices have been widely used in the medical diagnosis field despite having lower reliability than the large-scale equipment.

The platforms used in the POCT devices use a dry reagent that reacts with a specific substance and are classified into strip and cartridge types according to the type of measurement platform. The strip type is a method that uses glass fiber or a porous membrane to separate red blood cells from blood and simultaneously measures a change in color according to concentration, and the cartridge type is a method that measures serum (plasma) whose color is changed according to the concentration of a specific substance by allowing serum (plasma), from which red blood cells have been separated, to react with a dry reagent.

European patent application EP0799896 discloses a test strip having a matrix comprising a polysulfone membrane.

The present invention provides a strip-type platform for use in a POCT device and relates to a technique of removing red blood cells from blood using a porous membrane used in fabrication of a strip and simultaneously changing a color according to the concentration of a reactant (using a chemical colorimetric assay).

In the case of the strip-based platform, a technique of separating red blood cells from blood and a technique of specifically reacting a specific substance present in plasma from which red blood cells have been separated are used to measure a specific substance in blood. In particular, to remove red blood cells from plasma, a method of removing red blood cells by overlapping several (at least two) sheets of pads made of glass fiber and the like is commonly used. This method has good red blood cell separation efficiency, but it is painful and has difficulty in collection in the blood collection process because the use of a lot of blood is required.

Meanwhile, as a conventional technique of separating red blood cells and performing measurement at the same time using a porous membrane in a strip, there is a method using an anisotropic membrane proposed by LifeScan, Inc. in 1995 (US005789255A). This technique is characterized in that an upper layer part and a lower layer part have mutually different pore sizes, and measurement is performed simultaneously with the separation of red blood cells. However, the technique proposed by LifeScan, Inc. has a problem in which, in the case of people with a high red blood cell content (Hct%), red blood cells are not properly filtered in an upper layer part or are broken to cause a lower layer part to be stained, thereby resulting in poor accuracy and poor reproducibility of measurements.

### [Disclosure]

### [Technical Problem]

The present invention is directed to providing a strip-type platform for use in a point-of-care test (POCT) device, which increases the separation efficiency of a substance contained in a sample and improves the accuracy and reproducibility of measurement even with a small amount of sample.

Furthermore, the present invention is directed to providing a means which is able to be used to obtain information on a sample using the light source and camera of a portable terminal.

### [Technical Solution]

One aspect of the present invention provides a biomaterial assay strip which includes a porous matrix having micropores and composed of materials comprising a copolymer selected from the group comprising polysulfone, polyethersulfone, and polyarylsulfone, wherein the matrix includes an enzyme , a dye, and a hydrophilic polymer material, all of which react with a biomaterial, therein, the matrix is brought into contact with a biomaterial-containing sample in an upper layer thereof to measure the biomaterial contained in the sample, which is diffused toward a lower layer of the matrix, through color development in the matrix, and the strip includes an upper layer part where the average size of the micropores gradually decreases from the upper layer of the matrix up to 1/2 to 9/10 of a thickness, a separation part where the average size of the micropores is kept constant, and a lower layer part where the average size of the micropores gradually increases up to the lower layer of the matrix.

Preferably, the matrix has a thickness of 100 to 240 µm.

Preferably, the micropores have an average size of 0.2 to 0.6 µm.

Preferably, in the upper layer part, substances other than the biomaterial contained in the sample are filtered.

Preferably, substances other than the biomaterial and the biomaterial are structurally separated by the separation part in the strip, and the separation part acts like a mirror to increase the reflectance of light reflected by a surface.

Preferably, in the lower layer part, an enzymatic reaction of the biomaterial and color development caused by the dye occur.

Preferably, the color development occurs from 0.5 to 3 µl of a sample.

Preferably, the hydrophilic polymer material is polyethylene glycol.

Preferably, the polyethylene glycol has an average molecular weight ranging from 200 to 8,000.

Preferably, the dye reacts with the biomaterial contained in the sample or a substance produced by a reaction of the biomaterial.

Preferably, the dye is one or more selected from the group consisting of WST-4, WST-8, WST-9, DA67, new Trinder's reagents, and tetrazolium salts.

Preferably, the color development in the matrix is photographed by the camera of a portable terminal.

Preferably, the color development in the matrix corrects a value measured in the R region (530 to 675 nm) of the visible spectrum with a value measured in the G region (465 to 565 nm).

### [Advantageous Effects]

The present invention can provide a strip capable of increasing measurement efficiency, accuracy, and reproducibility in a point-of-care test (POCT) device. Accordingly, the present invention makes it possible to more simply and quickly obtain information on a specific substance in blood and information such as disease information, health information, and the like. In addition, when the strip of the present invention is used for measurement in a portable terminal, the utility thereof can be expanded.

### [Description of Drawings]

FIG. 1 is a scanning electron microscope (SEM) image illustrating the cross section of a strip according to an embodiment of the present invention.
FIG. 2 is an example of the cross-sectional structure of a strip according to the present invention.
FIG. 3 shows results measured on the day of fabrication of strips according to Example and Comparative Examples 1 and 2.
FIG. 4 shows results measured 7 days after fabrication of strips according to Example and Comparative Examples 1 and 2.

### [Modes of the Invention]

The present invention relates to a strip-type platform for use in a point-of-care test (POCT) device, and specifically, to a biomaterial assay strip which includes a porous matrix having micropores and composed of materials comprising a copolymer selected from the group comprising polysulfone, polyethersulfone, and polyarylsulfone. The matrix includes a hydrophilic polymer material, an enzyme that reacts with a biomaterial, or a dye that reacts with the biomaterial or a substance produced by a reaction of the biomaterial. The strip is brought into contact with a biomaterial-containing sample in an upper layer of the matrix to measure the biomaterial contained in the sample, which is diffused toward a lower layer of the matrix, through color development in the matrix. Also, the strip includes an upper layer part where the average size of the micropores gradually decreases from the upper layer of the matrix up to 1/2 to 9/10 of a thickness, a separation part where the average size of the micropores is kept constant, and a lower layer part where the average size of the micropores gradually increases up to the lower layer of the matrix.

The biomaterial is contained in body fluids (e.g., blood, saliva, urine, sweat, etc.), and information on disease or health may be obtained by measuring the biomaterial. Preferably, according to the present invention, when a sample is blood, information on at least one of blood sugar, cholesterol, cancer markers, liver levels, lung levels, triglycerides, potassium, uric acid, calcium-accepting protein (Troponon), CRP, NT-proBNP, and creatinine kinase may be obtained from a biomaterial in the blood to determine the presence or absence of a disease and the degree of progression thereof.

In the strip of the present invention, the porous matrix preferably has a thickness of 100 to 240 µm. This thickness allows a small amount of biomaterial-containing sample to be used. That is, when the thickness is more than 240 µm, the amount of a sample for measurement needs to increase. For example, when a sample is blood, a large amount of blood needs to be collected, which may impart pain to the subject. Therefore, the present invention enables accurate and reproducible measurement even with a small amount of sample by proposing the above-described thickness range. In the present invention, the amount of sample required for measurement is 0.5 to 3 µl.

The micropores distributed inside the matrix of the strip according to the present invention have an average size of 0.2 to 0.6 um.

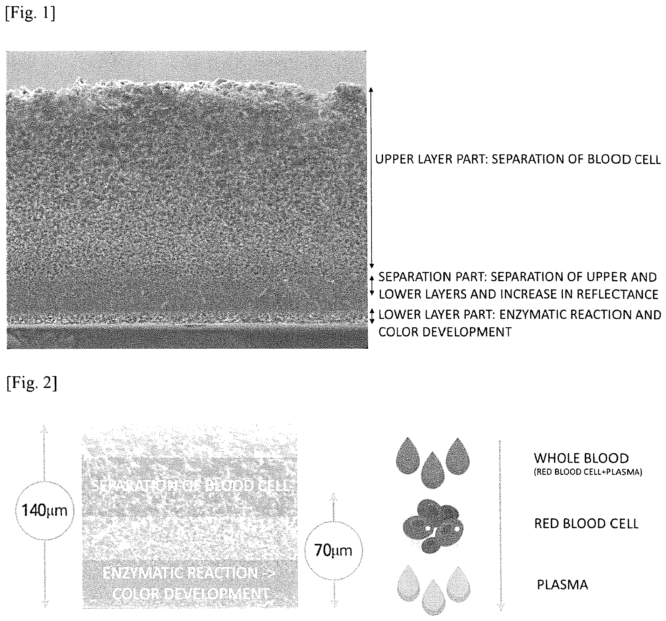

FIG. 1 is a photographic image illustrating the cross section of a strip according to an embodiment of the present invention, and FIG. 2 shows the cross-sectional structure of the strip of the present invention and specifically shows that when blood is used as a sample, blood cells and plasma are separated in an upper layer part, and color development occurs in a lower layer part.

As shown in FIGS. 1 and 2, it is confirmed that the micropores distributed inside the matrix of the present invention have varying sizes depending on the thickness or part of the matrix. That is, there are both a part where small-sized micropores are densely distributed and a part where large-sized micropores are distributed. Also, in some parts, it seems that micropores are hardly distributed due to the very small size of micropores.

In an embodiment, the micropores distributed in the matrix of the present invention have an average size gradually decreased in a part from the upper layer of the matrix up to 1/2 to 9/10 of a thickness. Next, a part where micropores have a constant average size is present. Then, micropores have an average size increased up to the lower layer. The micropores serve to filter impurities in a sample, for example, red blood cells when the sample is blood, to separate the same from plasma. That is, the separation of blood cells begins quickly due to large-sized micropores in the upper layer part and is gradually completed by micropores whose size becomes smaller in the downward direction. Subsequently, while passing through the part where micropores with a constant size are distributed, plasma and blood cells are completely separated, and then the size of micropores increases in the lower layer part to promote diffusion of plasma. In this case, a reaction between a biomaterial contained in a sample and an enzyme and color development caused by a dye are effectively achieved.

Therefore, the separation occurs in the upper layer part corresponding to 1/2 to 9/10 of the thickness of the matrix after a sample is brought into contact with the upper layer of the matrix, and the enzymatic reaction of a biomaterial and the color development caused by a dye preferably occur in the lower layer part of the matrix.

However, it is not easy to completely remove blood cells in the separation process because the size, amount, and shape of blood cells vary from person to person, and the shape of blood cells is likely to be distorted during the process of filtering the blood cells. For this reason, the operation for adjusting the shape or size of micropores was repeated several times, and the related art such as US Patent No. 7,125,493 proposed various methods such as adjusting a dope solution used for preparation of a matrix and the like, but these methods did not yield desired results. That is, it was confirmed that the strip as proposed in the US Patent may obtain reliable information on a biomaterial to some extent when at least two sheets are used. However, in the case of the present invention, satisfactory measurement results can be obtained with just one sheet of a strip having a thickness of 100 to 240 µm or less.

The strip of the present invention is formed so that a hydrophilic polymer material is included in the matrix. The hydrophilic polymer material helps a sample to be adsorbed onto the strip and allows the separation process to be effectively achieved. Preferably, the hydrophilic polymer material is polyethylene glycol (PEG). When PEG is used as the hydrophilic polymer material, it can be seen that the accuracy and reliability of measurement of the strip are improved as compared to when using other hydrophilic polymer materials. This is thought to be due to not only having the hydrophilicity of PEG but also having a residue particularly advantageous for the structural separation process, for example, for the adsorption of blood cells in the case of blood. Preferably, the PEG has an average molecular weight ranging from 200 to 8,000. This molecular weight range is preferred because it has been confirmed that accurate and reproducible measurement of a biomaterial is possible even through the strip of the present invention has a thin thickness of 240 µm or less. That is, when PEG having a molecular weight above or below the above-describe range is applied, a large amount of sample is required, or the time required to obtain information is delayed. Accordingly, the strip of the present invention enables measurement even with a small amount, specifically, 0.5 to 3 µl of a sample.

When a blood sample is added dropwise to the strip of the present invention which has been treated with a hydrophilic polymer material and compounds (enzyme and dye) that react with a specific biomaterial to cause color development, a rate of separating blood cells and plasma (serum) increases by the micropores distributed inside the matrix and the hydrophilic polymer material. The plasma (serum) separated from blood shows color development due to a compound (i.e., an enzyme or a dye) that reacts with a biomaterial, and a measured value may be obtained by converting the intensity of color developed in the lower layer of the matrix to a concentration.

The enzyme included in the matrix of the strip according to the present invention may react with a biomaterial, for example, in blood, such as blood sugar, cholesterol, neutral fats, uric acid, total protein, albumin, or the like, and furthermore, may react with a substance produced by a reaction of the biomaterial.

As an example, when blood sugar is measured, an enzyme required for the following reaction may be included.

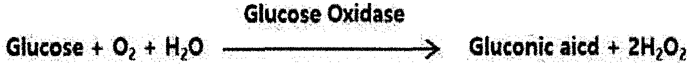

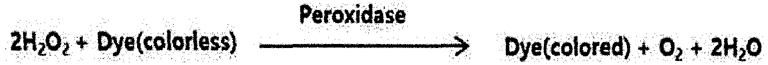

Meanwhile, as shown in the above reaction scheme, the dye included in the matrix of the present invention reacts with a biomaterial or a substance produced by a reaction of the biomaterial. The dye may be one or more selected from the group consisting of WST-4, WST-8, WST-9, DA67, new Trinder's reagents, and tetrazolium salts. The dye selected as described above shows an optimal result on the algorithm for obtaining information on a biomaterial from the strip of the present invention, which will be described below.

The strip of the present invention obtains information on a biomaterial in a sample using a value measured with light in the R region of the visible spectrum and a value measured with light in the G region of the visible spectrum. Information on a biomaterial may be obtained by light in the R region of the visible spectrum, and information on substances other than the biomaterial may be obtained by light in the G region of the visible spectrum. Therefore, when the information on a biomaterial in the R region is corrected with the value in the G region, more accurate measurement values may be obtained. Such a measurement algorithm may be realized by the size distribution of micropores in the strip of the present invention as described above.

That is, since substances other than the biomaterial in a sample are present in the upper layer part (from the upper layer up to 1/2 to 9/10 of a thickness) of the strip of the present invention, the amount thereof is measured with light in the G region of the visible spectrum. Also, color development caused by a biomaterial in the lower layer part of the strip is measured with light in the R region of the visible spectrum, and the intermediate separation part where the size of micropores is constant serves to structurally separate the upper layer part and the lower layer part and further acts like a mirror to increase the reflectance of light reflected by a surface, thereby minimizing interference caused by the upper layer part during measurement and obtaining more accurate information on a biomaterial.

In addition, it is confirmed that one or more dyes selected from the group consisting of WST-4, WST-8, WST-9, DA67, new Trinder's reagents, and tetrazolium salts as described above may provide accurate information on a biomaterial in a sample, particularly, as measured using the R region and G region of the visible spectrum as described in the present invention.

In order to provide accurate information on a biomaterial in a sample despite using a thinner strip thickness and a smaller amount of sample compared to the related art, the present invention provides the limited size distribution of micropores in the strip and the limited type of dye and also provides an algorithm for obtaining information from the strip.

Hereinafter, the present invention will be described with reference to examples. However, the present invention is not limited to the following examples.

### Example

### (Preparation of porous matrix)

Sample No. 1: 4 kg of a polyethersulfone resin as a polymer resin, 25 kg of dimethylformamide as a solvent, 15 kg of p-toluenesulfonic acid as a coagulation accelerator, and 6 kg of polyvinylpyrrolidone as a swelling agent were input into a dissolution bath, dissolved at 65 °C, and then cooled to 40 °C. The resulting solution was charged with nitrogen, a pressure was reduced to a vacuum state for at least 12 hours to sufficiently remove air bubbles inside the solution, and the resulting solution was transferred to a casting plate. A 2 m-wide polyester film was used as a supporting layer, the solution was passed so that the width of the solution became 0.75 m with the casting plate adjusted so that the gap between a casting knife and a polyester film surface was 140 um, and then the resultant was immersed in a coagulation bath consisting 5 °C water. After confirming the sufficient solidification of the solution in the coagulation bath, the resultant was transferred to a washing bath set at 65 °C. One minute after the immersion in the washing bath, the polyethersulfone membrane and the polyester film as a supporting layer were detached, and washing was performed again in water for 20 minutes. After the washing was completed, water remaining on a porous matrix surface was removed using an air knife, and drying was performed in a dryer to prepare a porous polyethersulfone matrix.

Sample No. 2: A porous matrix having the average size and distribution of micropores as shown in Table 1 was prepared in the same manner as in the above method, except that 5 kg of a polyethersulfone resin as a polymer resin and 5 kg of polyvinylpyrrolidone as a swelling agent were adjusted.

Sample No. 3: A porous matrix having the average size and distribution of micropores as shown in Table 1 was prepared in the same manner as in the above method, except that 6 kg of a polyethersulfone resin as a polymer resin and 4 kg of polyvinylpyrrolidone as a swelling agent were adjusted.

**[Table 1]**

| Sample No. | Average size of micropore (µm) | Average size distribution of micropore | Matrix material | Thickness (µm) |
|---|---|---|---|---|
| 1 | 0.6 | 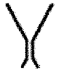 | PES | 140 |
| 2 | 0.4 | 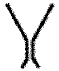 | PES | 140 |
| 3 | 0.292 | 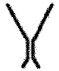 | PES | 140 |

### (Fabrication of strip)

As a porous matrix used to fabricate a strip, Sample No. 1 shown in Table 1 was used. A reagent to be treated on a porous matrix was prepared by quantitatively dissolving 16.9 kU of glucose oxidase, 13.3 kU of horseradish peroxide, 10 mg of DA67, and 20% PEG 6000 (average molecular weight: 6000) in 10 ml of a 20 mM MES buffer solution. After the complete dissolution of the reagent, a 20 mM MES buffer solution was further added so that a final volume became 15 ml. The porous matrix was immersed in the prepared reagent, taken out when complete absorption was achieved, and dried in an oven set at 40 °C for 30 minutes to fabricate a strip.

### Comparative Example 1

A strip was fabricated in the same manner as in Example, except that the addition of 20% PEG 6000 (average molecular weight: 6000) was omitted.

### Comparative Example 2

A strip was fabricated in the same manner as in Example, except that 1% PVP 20000 (average molecular weight: 20000) was added instead of adding 20% PEG 6000 (average molecular weight: 6000)

The strips fabricated in Example and Comparative Examples 1 and 2 were subjected to the following experiment.

### Test method

A blood sample was prepared at a glucose concentration of 50 mg/dL using standard equipment (Cobas^{®}8000). A commercially available glucose (Sigma-Aldrich Inc.) reagent was added to the blood sample so that the concentration became 100, 150, 200, 250, 300, and 350 mg/dL.

The blood sample was injected three times in an amount of 1 ul into each of the strips fabricated in Example and Comparative Examples 1 and 2, and an average value was recorded.

One week later, a blood sample was prepared by the above method, and the same experiment was performed.

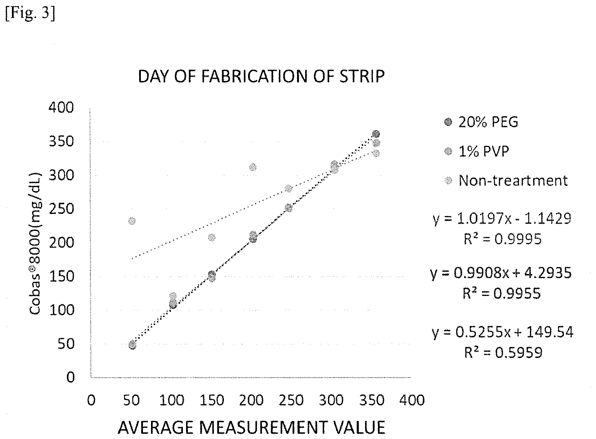

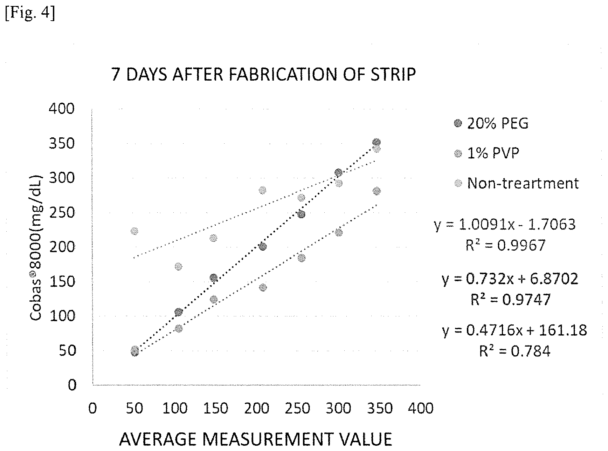

Experimental results are shown in FIGS. 3 and 4. FIG. 3 shows a result measured on the day of fabrication of the strip, and FIG. 4 shows a result measured 7 days after fabrication.

Looking at the results, the strip of Comparative Example 1 showed a significant difference from the strips of Example and Comparative Example 2 even on the day of fabrication. Also, there was no significant difference between the strips of Example and Comparative Example 2 on the day of fabrication, but the difference was shown at 7 days after fabrication. That is, the strip of the present invention maintained its measurement performance even when time elapsed, but the strip of Comparative Example 2 clearly exhibited degraded measurement performance over time.

## Claims

1. A biomaterial assay strip comprising a porous matrix having micropores and composed of materials comprising a copolymer selected from the group comprising polysulfone, polyethersulfone, and polyarylsulfone,
wherein the matrix includes an enzyme, a dye, and a hydrophilic polymer material, all of which react with a biomaterial, therein,
the matrix is brought into contact with a biomaterial-containing sample in an upper layer thereof to measure the biomaterial contained in the sample, which is diffused toward a lower layer of the matrix, through color development in the matrix,
there are an upper layer part where the average size of the micropores gradually decreases from the upper layer of the matrix up to 1/2 to 9/10 of a thickness, a separation part where the average size of the micropores is kept constant, and a lower layer part where the average size of the micropores gradually increases up to the lower layer of the matrix, and
the color development occurs from 0.5 to 3 µl of a sample.

2. The biomaterial assay strip of claim 1, wherein the matrix has a thickness of 100 to 240 µm.

3. The biomaterial assay strip of claim 1, wherein the micropores have an average size of 0.2 to 0.6 µm.

4. The biomaterial assay strip of claim 1, wherein, in the upper layer part, substances other than the biomaterial contained in the sample are filtered.

5. The biomaterial assay strip of claim 1, wherein the upper layer part and lower layer part in the strip are structurally separated by the separation part, and the separation part acts like a mirror to increase the reflectance of light reflected by a surface.

6. The biomaterial assay strip of claim 1, wherein, in the lower layer part, an enzymatic reaction of the biomaterial and color development caused by the dye occur.

7. The biomaterial assay strip of claim 1, wherein the hydrophilic polymer material is polyethylene glycol.

8. The biomaterial assay strip of claim 7, wherein the polyethylene glycol has an average molecular weight ranging from 200 to 8,000.

9. The biomaterial assay strip of claim 1, wherein the enzyme reacts with a biomaterial in blood and a substance produced by a reaction of the biomaterial.

10. The biomaterial assay strip of claim 1, wherein the dye reacts with a biomaterial or a substance produced by a reaction of the biomaterial.

11. The biomaterial assay strip of claim 1, wherein the dye is one or more selected from the group consisting of WST-4, WST-8, WST-9, DA67, new Trinder's reagents, and tetrazolium salts.

12. The biomaterial assay strip of claim 1, wherein information on the biomaterial is obtained by correcting a value measured in the R region (530 to 675 nm) of the visible spectrum with a value measured in the G region (465 to 565 nm).

## Patentansprüche

1. Biomaterialteststreifen, umfassend eine poröse Matrix mit Mikroporen, die aus Materialien gebildet ist, die ein aus der Polysulfon, Polyethersulfon und Polyarylsulfon umfassenden Gruppe ausgewähltes Copolymer umfassen,
wobei die Matrix ein Enzym, einen Farbstoff und ein hydrophiles Polymermaterial aufweist, die jeweils mit einem Biomaterial reagieren, wobei die Matrix in einer oberen Schicht mit einer Biomaterial enthaltenden Probe in Kontakt bringbar ist, um durch eine Farbentwicklung in der Matrix das in der Probe enthaltene Biomaterial zu messen, das in Richtung einer unteren Schicht der Matrix diffundiert,
wobei ein oberer Schichtabschnitt, in dem die mittlere Größe der Mikroporen ausgehend von der oberen Schicht der Matrix graduell bis zu 1/2 bis 9/10 einer Dicke abnimmt, ein Trennungsabschnitt, in dem die mittlere Größe der Mikroporen konstant ist, und ein unterer Schichtabschnitt, in dem die mittlere Größe der Mikroporen bis zu der unteren Schicht der Matrix graduell zunimmt, vorgesehen sind und die Farbentwicklung ab 0,5 µl bis 3 µl einer Probe stattfindet.

2. Biomaterialteststreifen nach Anspruch 1, wobei die Matrix eine Dicke von 100 µm bis 240 µm aufweist.

3. Biomaterialteststreifen nach Anspruch 1, wobei die Mikroporen eine mittlere Größe von 0,2 µm bis 0,6 µm aufweisen.

4. Biomaterialteststreifen nach Anspruch 1, wobei in dem oberen Schichtabschnitt Substanzen, bei denen es sich nicht um das in der Probe enthaltene Biomaterial handelt, herausfilterbar sind.

5. Biomaterialteststreifen nach Anspruch 1, wobei der obere Schichtabschnitt und der untere Schichtabschnitt in dem Streifen durch den Trennungsabschnitt strukturell voneinander getrennt sind und der Trennungsabschnitt nach Art eines Spiegels wirkt, so dass der Reflexionsgrad von durch eine Fläche reflektiertem Licht erhöht ist.

6. Biomaterialteststreifen nach Anspruch 1, wobei in dem unteren Schichtabschnitt eine enzymatische Reaktion des Biomaterials und eine durch den Farbstoff hervorgerufene Farbentwicklung stattfinden.

7. Biomaterialteststreifen nach Anspruch 1, wobei es sich bei dem hydrophilen Polymermaterial um Polyethylenglycol handelt.

8. Biomaterialteststreifen nach Anspruch 7, wobei das Polyethylenglycol ein mittleres Molekulargewicht von 200 bis 8000 aufweist.

9. Biomaterialteststreifen nach Anspruch 1, wobei das Enzym mit einem Biomaterial in Blut und einer durch eine Reaktion des Biomaterials erzeugten Substanz reagiert.

10. Biomaterialteststreifen nach Anspruch 1, wobei der Farbstoff mit einem Biomaterial oder einer durch eine Reaktion des Biomaterials erzeugten Substanz reagiert.

11. Biomaterialteststreifen nach Anspruch 1, wobei es sich bei dem Farbstoff um einen oder mehrere aus der WST-4, WST-8, WST-9, DA67, neue Trinder-Reagenzien und/oder Tetrazoliumsalze umfassenden Gruppe ausgewählten Farbstoff bzw. ausgewählte Farbstoffe handelt.

12. Biomaterialteststreifen nach Anspruch 1, wobei Informationen zu dem Biomaterial durch Korrigieren eines in der R-Region (530 nm bis 675 nm) des sichtbaren Spektrums gemessenen Werts mit einem in der G-Region (465 nm bis 565 nm) gemessenen Wert ermittelbar sind.

## Revendications

1. Bande d'analyse de biomatériau comprenant une matrice poreuse ayant des micropores et composée par des matériaux comprenant un copolymère sélectionné dans le groupe consistant en polysulfone, polyéthersulfone et polyarylsulfone, dans laquelle la matrice comporte un enzyme, un colorant et un matériau polymère hydrophile, dont chacun réagit avec un biomatériau, dans laquelle la matrice est mise en contact avec un échantillon contenant du biomatériau dans une couche supérieure de celle-ci afin de mesurer le biomatériau contenu dans l'échantillon, qui est diffusé vers une couche inférieure de la matrice, par l'intermédiaire d'un développement de couleur dans la matrice,
il y a une partie de couche supérieure où la taille moyenne des micropores abaisse graduellement à partir de la couche supérieure de la matrice jusqu'à 1/2 à 9/10 d'une épaisseur, une partie de séparation où la taille moyenne des micropores est constante et une partie de couche inférieure où la taille moyenne des micropores augmente graduellement jusqu'à la couche inférieure de la matrice et
le développement de couleur se produit de 0,5 µl à 3 µl d'un échantillon.

2. Bande d'analyse de biomatériau selon la revendication 1, dans laquelle la matrice a une épaisseur de 100 µm à 240 µm.

3. Bande d'analyse de biomatériau selon la revendication 1, dans laquelle les micropores ont une taille moyenne de 0,2 µm à 0,6 µm.

4. Bande d'analyse de biomatériau selon la revendication 1, dans laquelle, dans la partie de couche supérieure, des substances autre que le biomatériau contenu dans l'échantillon sont filtrés.

5. Bande d'analyse de biomatériau selon la revendication 1, dans laquelle la partie de couche supérieure et la partie de couche inférieure dans la bande sont séparées structurellement par la partie de séparation et la partie de séparation fonctionne comme un miroir pour augmenter la réflectance de la lumière réfléchie par une surface.

6. Bande d'analyse de biomatériau selon la revendication 1, dans laquelle, dans la partie de couche inférieure, une réaction enzymatique du biomatériau et le développement de couleur causé par le colorant se produisent.

7. Bande d'analyse de biomatériau selon la revendication 1, dans laquelle le matériau polymère hydrophile est le polyéthylène glycol.

8. Bande d'analyse de biomatériau selon la revendication 7, dans laquelle le polyéthylène glycol a un poids moléculaire moyen de 200 à 8000.

9. Bande d'analyse de biomatériau selon la revendication 1, dans laquelle l'enzyme réagit avec un biomatériau dans le sang et une substance produite par une réaction du biomatériau.

10. Bande d'analyse de biomatériau selon la revendication 1, dans laquelle le colorant réagit avec un biomatériau ou une substance produite par une réaction du biomatériau.

11. Bande d'analyse de biomatériau selon la revendication 1, dans laquelle le colorant est l'un ou plusieurs sélectionnés dans le groupe consistant en WST-4, WST-8, WST-9, DA67, nouveaux réactifs de Trinder et sels de tétrazolium.

12. Bande d'analyse de biomatériau selon la revendication 1, dans laquelle des informations sur le biomatériau sont obtenues en corrigeant une valeur mesurée dans la région R (530 nm à 675 nm) du spectre visible par une valeur mesuré dans la région G (465 nm à 565 nm).
